# EUROPÄISCHE PATENTSCHRIFT

(11) **EP 1 452 141 B1**
(45) Veröffentlichungstag und Bekanntmachung des Hinweises auf die Patenterteilung: **14.04.2010**
(21) Anmeldenummer: 04004334.1
(22) Anmeldetag: 26.02.2004
(51) Int. Cl.: A61B 17/22, B06B 1/06, G10K 15/04

(54) **Vorrichtung zur Erzeugung von Stosswellen**
Shock wave generating device
Dispositif de génération d'ondes de choc

(30) Priorität: 26.02.2003 CH 2992003
(43) Veröffentlichungstag der Anmeldung: 01.09.2004
(73) Patentinhaber: Sanuwave, Inc., Alpharetta, GA 30022 (US)
(72) Erfinder: Simnacher, Erwin, 78479 Reichenau (DE)
(74) Vertreter: von Eichel-Streiber, Caspar

(56) Entgegenhaltungen:
- EP-A- 0 351 015
- EP-A- 0 436 809
- WO-A-99/48621
- US-A- 4 858 597
- US-A- 5 111 805
- US-A- 5 950 291
- US-A1- 2002 063 495

## Beschreibung

Die Erfindung betrifft ein Vorrichtung zur Erzeugung von Stosswellen gemäss dem Oberbegriff des Anspruchs 1.

Stosswellen werden in der Human- und Veterinärmedizin für unterschiedliche Zwecke eingesetzt. Eine medizinische Anwendung dieser Vorrichtungen in der Humanmedizin liegt in der Lithotripsie, bei der die erzeugten Stosswellen auf zu zerstörende innere Objekte, wie Nierensteine fokussiert werden. Weitere Anwendungen bestehen beispielsweise in der Induzierung von Knochenwachstum, der Behandlung von orthopädisch schmerzhaften Erkrankungen (Epicondylitis, Kalkschulter) und der Behandlung von Nerven, Muskeln und anderen Weichteilstrukturen.

Die Erzeugung von Stosswellen unter Verwendung piezoelektrischer Keramikelemente ist grundsätzlich bekannt, beispielsweise aus EP 0436 809 A2.
Eine Vielzahl von piezoelektrischen Keramikelementen sind an einer Kugelkalotte angeordnet und bilden einen elektroakustischen Wandlern.

Die Anordnung dieser Vielzahl von piezoelektrischen Keramikelementen der bekannten Vorrichtung ist sehr aufwendig und kostenintensiv in der Herstellung.

Die piezoelektrischen Keramikelemente sind in einer Vergussmasse aus beispielsweise einem Epoxydharzgemisch eingebettet.
Da die Abstrahlfläche der piezoelektrischen Keramikelemente eine Fläche von mehreren Quadratmillimetern bis zu einigen Quadratzentimetern umfasst, führt die Deformation der piezoelektrischen Keramikelemente zu einer starken Beanspruchung der Vergussmasse an der Grenzschicht zu den Keramikelementen.

Generell ist man bestrebt, die Bauform der Stosswellenerzeugungsvorrichtungen zu miniaturisieren. Dieses Ziel wird verfolgt um einerseits die Handhabung der Geräte zu vereinfachen und andererseits neue Anwendungsbereiche beispielsweise für die Behandlung von Speichelsteinen zu erschliessen.

Zudem ist es wünschenswert die Stosswellen auf Bereiche unterschiedlicher Geometrie zu fokussieren. Hierzu sind frei wählbare Geometrieformen der Stosswellenerzeugungssysteme erforderlich. Damit wird eine hohe Effizienz bei Spezialanwendungen erreicht, beispielsweise für die Behandlung von langen Knochenspalten oder Zellulitis.

Vor dem Hintergrund der vorstehenden Ausführung liegt der Erfindung die Aufgabe zugrunde, eine Vorrichtung zur Erzeugung von Stosswellen der genannten Gattung zu schaffen, die einfach und kostengünstig herzustellen ist, zuverlässig in der Anwendung und zudem hinsichtlich der Baugrösse flexibler gestaltet werden kann.

Diese Aufgabe wird erfindungsgemäss gelöst durch eine Vorrichtung mit den Merkmalen des Anspruchs 1.

Vorteilhafte Ausführungen der Erfindung sind in den Unteransprüchen angegeben.

Der wesentliche Gedanke der Erfindung besteht darin, piezoelektrische Fasern, nachfolgend Piezofasern genannt, für die Erzeugung von Stosswellen zu verwenden. Die in einem Verbundwerkstoff eingebrachten Piezofasern werden dazu angesteuert und bilden zusammen mit einer Ansteuereinheit den die Stosswellen erzeugenden Teil.

Für die Erzeugung von Stosswellen wird der indirekte piezoelektrische Effekt der Fasermaterialien genutzt.

Ein äußeres elektrisches Feld übt auf die positiv und negativ geladenen Ionen im Kristallgitter entgegen gerichtete Kräfte aus. Hierbei kommt es zu einer Deformation der Fasermaterialien. Die Piezofasern dehnen sich dabei hauptsächlich in ihrer Längsrichtung aus.

Dies kurze Ausdehnung kann zur Stoßwellenerzeugung genutzt werden. Dies is aus der Wo 99/48621 gemäss dem Oberbegriff des ersten Anspruchs, bekannt.

Erfindungsgemäß sind alle Piezofasern in dem Verbundwerkstoff derart eingebracht, dass ihr Längsrichtung auf das zu behandelnde Gebiet und/oder in Ausbreitungsrichtung der Stosswelle weist. Dadurch kann eine hohe Energiedichte im Fokusbereich erreicht werden.

Die Piezofasern lassen sich einfach und gleichmäßig verteilt in den Werkstoff einbetten. Die Verbindung der Piezofasen mit dem Verbundwerkstoff ist damit homogen.

Die Kontaktierung der Piezofasern kann entsprechend den Verschaltungsanforderungen durch eine gemeinsame elektrisch leitende Schicht erfolgen. Dadurch entfällt die aufwendige Verschaltung der Vielzahl von piezoelektrischen Keramikelementen der bekannten elektroakustischen Wandler.

Die in den Verbundwerkstoff eingebrachten Piezofasern bilden mit dem Verbundwerkstoff mindestens ein Modul.

Dieses mindestens eine Modul kann in einer bevorzugten Ausführungsform der erfindungsgemässen Vorrichtung eine räumliche Einheit bildet. Es ist aber auch denkbar, das das mindestens eine Modul mittels gemeinsam elektrisch verbundener Piezofasern eine Einheit bildet.

Weiterhin lassen sich die Piezofasern in gekrümmte Strukturen bringen. In beiden vorweg genannten Ausführungsvarianten kann somit das mindestens eine Modul in geometrisch' verschiedenen Formen ausgeführt sein.

Dies ermöglicht eine hohe Flexibilität in der Ausführungsform der Stosswellenerzeugungsvorrichtung. Es lassen sich somit Vorrichtungen zur Erzeugung von Stosswellen verschiedener geometrischer Formen realisieren.

Zudem können mehrere Module benachbart angeordnet werden. Die Verschaltung der Module kann einzeln, in Gruppen oder miteinander erfolgen.

Um eine möglichst kompakte Anordnung der Stosswellenerzeugungsvorrichtung zu erreichen, ist das mindestens eine Modul vorzugsweise an einem Träger angeordnet.

Die einzelnen Piezofasern können in einer gesonderten Ausführungsvariante an den jeweiligen Anschlussenden gemeinsam kontaktiert ausgeführt sein. Ist der Modulträger elektrisch leitend ausgeführt, kann eine der beiden Kontaktierungen mit dem Modulträger verbunden sein.

Der Modulträger kann verschiedene geometrische Formen aufweisen.

Die vorweg genannte vorteilhafte Variation der erfindungsgemässen Vorrichtung zum Erzeugen von Stosswellen hinsichtlich der Geometrie gestattet die Möglichkeit einer Miniaturisierung der Vorrichtung. Dies ermöglicht die Herstellung von kleinen Stosswellenerzeugungsvorrichtungen der genannten Gattung für die intrakorporale Anwendungen.

Es können flächige Modulen mit in den Verbundwerkstoff eingebrachten Piezofasern bei beliebiger Formgebung hergestellt werden.

Für Spezialanwendungen lassen sich somit mit der erfindungsgemässen Vorrichtung nicht nur kleinere Stosswellenerzeugungsvorrichtungen realisieren, sondern auch flächige Stosswellenerzeugungsvorrichtungen mit verschiedenen Fokusgeometrien.

Im Folgenden wird die Erfindung anhand von in der Zeichnung dargestellten Ausführungsbeispielen näher erläutert. Es zeigen:
- Figur 1: eine Seiten- und Stirnansicht der erfindungsgemässen Vorrichtung in einer ersten Ausführung,
- Figur 2: eine Seiten- und Stirnansicht der erfindungsgemässen Vorrichtung in einer zweiten Ausführung und
- Figur 3: eine Stirnansicht der erfindungsgemässen Vorrichtung in einer Ausführung mit mehreren Modulen.

In den Zeichnungen sind einige Ausführungsbeispiele schematisch und vereinfacht dargestellt.

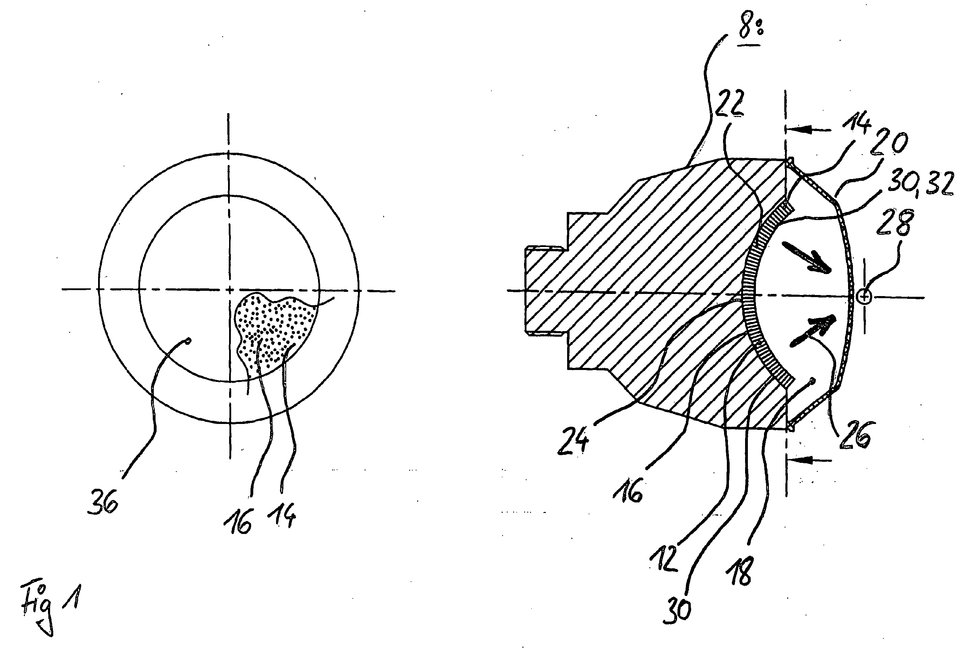

In Figur 1 ist eine Stosswellenerzeugungsvorrichtung 8 gezeigt, die einen die Stosswellen erzeugenden Teil 12 und ein für die Stosswellenübertragung geeignetes Medium 18 aufweist, welches ein Volumen zwischen dem Stosswellen erzeugenden Teil 12 und einer Koppelmembran 20 ausfüllt. Als für die Stosswellenübertragung geeignetes Medium 18 wird beispielsweise Wasser oder Gel verwendet. Die Koppelmembran 20 dient der energetisch verlustarmen Ankopplung der Stosswellenerzeugungsvorrichtung 8 an ein zu behandelndes Körperteil.

Die Stosswellen werden von dem die Stosswellen erzeugenden Teil 12 generiert und breiten sich in der dargestellten Richtung 26 aus.
Sie werden basierend auf der vorgegebenen Geometrie des die Stosswellen erzeugenden Teils 12 in einem Stosswellenfokus 28 gebündelt. Der Stosswellenfokus 28 ist der Bereich mit der grössten Energiedichte. In dem in Figur 1 gezeigten Ausführungsbeispiel ist der die Stosswellen erzeugende Teil 12 in Form eines Kugelsegmentes ausgebildet. Dies führt zu einer Fokussierung der Stosswelle. Die Fokussierung kann auch in bekannter und daher nicht näher zu beschreibender Weise elektronisch erfolgen.

Den die Stosswellen erzeugenden Teil 12 bilden in einen Verbundwerkstoff 16 eingebrachte Piezofasern 14. Die Piezofasern 14 sind an den jeweiligen Enden 30 elektrisch verschalten und werden mit einer Hochspannung beaufschlagt. Die Hochspannung wird dabei vorzugsweise impulsförmig angelegt.

Die Piezofasern 14 sind derart in den Verbundwerkstoff 16 eingebracht, dass sie vorzugsweise in Ihrer Längsrichtung in Ausbreitungsrichtung der Stosswellen 26 weisen, da sie sich hauptsächlich in dieser Richtung ausdehnen und somit den grössten Hub erzielen können. Diese kurze Ausdehnung der Piezofasern 14 wird in der erfindungsgemässen Vorrichtung zur Erzeugung von Stosswellen genutzt.

Werden nun die Piezofasern 14 mit einem Hochspannungsimpuls beaufschlagt, kommt es zur Ausdehnung der Piezofasern 14, die eine Stosswelle an einer Stirnfläche 32 der Piezofasern 14 entstehen lässt. Die erzeugte Stosswelle wird entsprechend der Geometrie des Stosswellen erzeugenden Teils 12 in einem Stosswellenfokus 28 gebündelt.

Der Verbundwerkstoff 16 bildet mit den eingebrachten Fasern 14 in dem dargestellten Ausführungsbeispiel eine räumliche Einheit, nachfolgend Modul 22 genannt. Das Modul 22 in der geometrischen Form eines Kugelsegmentes ist an einem Träger 24 angeordnet.

Die Piezofasern 14 sind an ihren jeweiligen Anschlussenden 30 gemeinsam kontaktiert ausgeführt und werden jeweils über Zuleitungen mit einer Ansteuervorrichtung, die hier nicht dargestellt ist, verbunden.

In einer besonders vorteilhaften Ausführung der erfindungsgemässen Vorrichtung ist das Modul 22 an einen elektrisch leitenden Modulträger 24 angeordnet, welcher elektrisch leitend mit einem von beiden Anschlüssen, die hier nicht dargestellt sind, der jeweils gemeinsam kontaktierten Anschlussenden 30 der Piezofasern 14 verbunden ist.

Wie vorweg bereits aufgeführt, kann mit der Formgebung des Modulträgers 24 und des Moduls 22 die Geometrie des Stosswellenfokus 28 bestimmt werden.

In dem dargestellten ersten Ausführungsbeispiel der Figur 1 wird ein Stosswellenfokus in der Form eines Ellipsoids erzeugt.

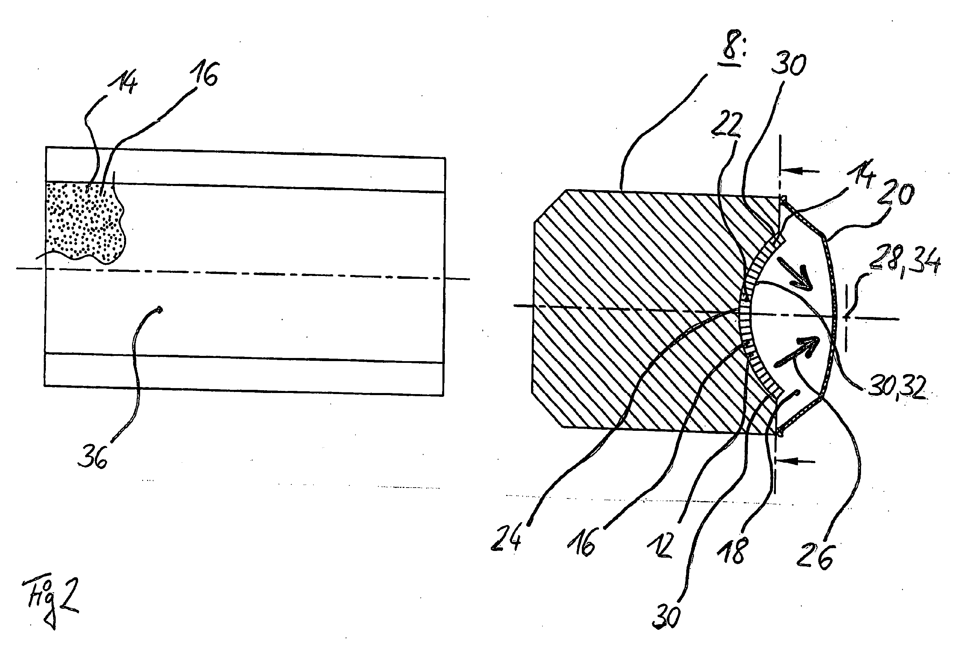

Im dargestellten zweiten Ausführungsbeispiel, welches in Figur 2 gezeigt ist, wird eine horizontale zylinderförmige Fokuslinie 34 erzeugt. Dazu ist der die Stosswellen erzeugende Teil 12 geometrisch in Form eines Rohrsegmentes ausgeführt.

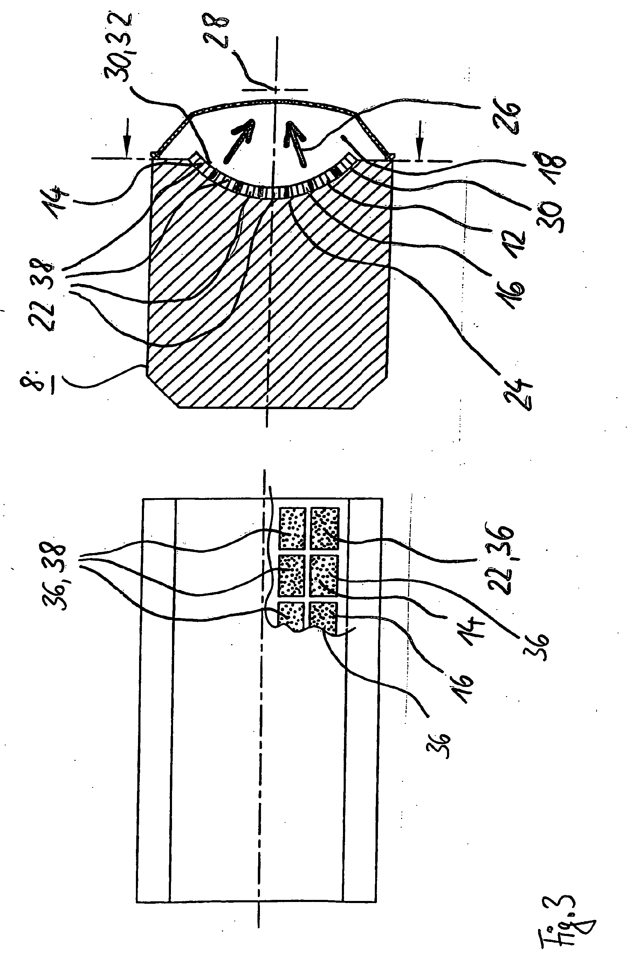

Gemäss einer bevorzugten Ausführungsform der Erfindung können, wie in Figur 3 dargestellt, mehrere Module 22 benachbart angeordnet sein. Die einzelnen Module 22 können dabei unterschiedlich gross und verschiedene Formen hinsichtlich ihrer Abstrahlungsfläche 36 aufweisen. Die Module 22 können einzeln angesteuert werden. Hiermit lässt sich beispielsweise eine gegenseitig verzögerte Ansteuerung der einzelnen Module 22 erreichen. Sie können aber auch in Modulgruppen 38 verschaltet und ansteuerbar sein.

Das Anlegen der Hochspannung erfolgt in bekannter Weise durch einen Hochspannungs-Impulsgenerator, dessen erster Pol an dem einen Anschlussende 30 der gemeinsam kontaktierten Piezofasern 14 und dessen zweiter Pol an dem anderen Anschlussende 30 der gemeinsam kontaktierten Piezofasern 14 angeschlossen wird.

In einer vorteilhaften Ausführung ist das an dem Modulträger 24 gemeinsame Anschlussende 30 der Piezofasern 14 mit dem vorzugsweise elektrisch leitenden Trägermaterial 24 verbunden. Der Modulträger 24 kann somit direkt an einen Pol des Hochspannungs-Impulsgenerators angeschlossen werden.

### Bezugszeichenliste

- 8: Stosswellenerzeugungsvorrichtung
- 12: Stosswellen erzeugender Teil
- 14: Piezofasern, piezoelektrische Fasern
- 16: Verbundwerkstoff
- 18: Für die Stosswellenausbreitung geeignetes Medium
- 20: Koppelmembran
- 22: Modul
- 24: Träger
- 26: Ausbreitungsrichtung der Stosswellen
- 28: Stosswellenfokus
- 30: Anschlussenden der gemeinsam kontaktierten Piezofasern
- 32: Stirnfläche der Piezofasern in Stosswellenausbreitungsrichtung
- 34: Fokuslinie
- 36: Modulabstrahlfläche
- 38: Modulgruppe

## Patentansprüche

1. Vorrichtung zur Erzeugung von Stosswellen, die auf ein zu behandelndes Gebiet eines menschlichen oder tierischen Körpers gerichtet sind, beinhaltend einen die Stosswellen erzeugenden Teil (12) aus in einen Verbundwerkstoff (16) eingebrachten piezoelektrischen Fasern (14) **dadurch gekennzeichnet, dass** alle piezoelektrischen Fasern (14) in dem Verbundwerkstoff (16) derart eingebracht sind, dass ihre Längsrichtung auf das zu behandelnde Gebiet und/oder in Ausbreitungsrichtung (26) der Stosswellen weist.

2. Vorrichtung nach einem der vorhergehenden Ansprüche,
**dadurch gekennzeichnet, dass** die in den Verbundwerkstoff (16) eingebrachten piezoelektrischen Fasern (14) mit dem Verbundwerkstoff (16) mindestens ein Modul (22) bilden.

3. Vorrichtung nach Anspruch 2,
**dadurch gekennzeichnet, dass** das mindestens eine Modul (22) eine räumliche Einheit bildet.

4. Vorrichtung nach Anspruch 2,
**dadurch gekennzeichnet, dass** das mindestens eine Modul (22) mittels gemeinsam elektrisch verbundener piezoelektrischer Fasern (14) eine Einheit bildet.

5. Vorrichtung nach einem der vorhergehenden Ansprüche,
**dadurch gekennzeichnet, dass** das mindestens eine Modul (22) in geometrisch verschiedenen Formen ausgeführt ist.

6. Vorrichtung nach einem der vorhergehenden Ansprüche,
**dadurch gekennzeichnet, dass** mehrere Module (22) benachbart angeordnet und einzeln, in Gruppen oder miteinander verschaltet sind.

7. Vorrichtung nach einem der vorhergehenden Ansprüche,
**dadurch gekennzeichnet, dass** das mindestens eine Modul (22) an einem Träger (24) angeordnet ist.

8. Vorrichtung nach Anspruch 7,
**dadurch gekennzeichnet, dass** der Modulträger (24) in geometrisch verschiedenen Formen ausgeführt ist.

## Claims

1. A device for generating shock waves, which are directed to a region to be treated of a human or animal body, containing a section (12) generating the shock waves, made of piezoelectric fibres (14) integrated into a composite material (16) **characterised in that** the piezoelectric fibres (14) are integrated into the composite material (16) in such a way that their longitudinal direction points out to the region to be treated and/or the direction of propagation (26) of the shock waves.

2. A device according to any of the preceding claims, **characterised in that** the piezoelectric fibres (14) integrated into the composite material (16) form at least one module (22) with the composite material (16).

3. A device according to claim 2, **characterised in that** said at least one module (22) forms a spatial unit.

4. A device according to claim 2, **characterised in that** said at least one module (22) forms a unit via piezoelectric fibres (14) electrically connected together.

5. A device according to any of the preceding claims, **characterised in that** said at least one module (22) may take on various geometric shapes.

6. A device according to any of the preceding claims, **characterised in that** several modules (22) arranged close to one another and individually, are linked in groups or with one another.

7. A device according to any of the preceding claims, **characterised in that** said at least one module (22) is arranged on a carrier (24).

8. A device according to claim 7, **characterised in** so that the module carrier (24) may take on various geometric shapes.

## Revendications

1. Dispositif de production d'ondes de choc, qui sont dirigées sur une zone à traiter d'un corps humain ou animal, contenant une partie (12) générant les ondes de choc partie constituée de fibres piézoélectriques (14) intégrées dans un matériau composite (16), **caractérisé en ce que** toutes les fibres piézoélectriques (14) sont intégrées au matériau composite (16) de telle sorte que leur direction longitudinale est dirigée vers la zone à traiter et/ou le sens de propagation (26) des ondes de choc.

2. Dispositif selon l'une quelconque des revendications précédentes, **caractérisé en ce que** les fibres piézoélectriques (14) intégrées au matériau composite (16) constituent avec le matériau composite (16) au moins un module (22).

3. Dispositif selon la revendication 2, **caractérisé en ce que** ledit au moins un module (22) constitue une unité spatiale.

4. Dispositif selon la revendication 2, **caractérisé en ce que** ledit au moins un module (22) constitue une unité à l'aide de fibres piézoélectriques (14) reliées électriquement ensemble.

5. Dispositif selon l'une quelconque des revendications précédentes, **caractérisé en ce que** ledit au moins un module (22) peut prendre diverses formes géométriques.

6. Dispositif selon l'une quelconque des revendications précédentes, **caractérisé en ce que** plusieurs modules (22) disposés les uns près des autres et individuellement, sont reliés en groupes ou les uns aux autres.

7. Dispositif selon l'une quelconque des revendications précédentes, **caractérisé en ce que** ledit au moins un module (22) est prévu sur un support (24).

8. Dispositif selon la revendication 7, **caractérisé en ce que** le support de module (24) peut prendre diverses formes géométriques.
